(19) Europäisches Patentamt
European Patent Office
Office européen des brevets

(11) Publication number: 0 200 746 B1

(12) # EUROPEAN PATENT SPECIFICATION

(45) Date of publication of patent specification: 30.01.91

(51) Int. Cl.[5]: **C 12 N 5/10,** C 12 N 15/82, A 01 H 1/00, C 12 P 19/34, C 07 H 21/04

(21) Application number: 85905144.3

(22) Date of filing: 26.09.85

(86) International application number: PCT/US85/01848

(87) International publication number: WO 86/02097 10.04.86 Gazette 86/08

(54) **PLANT CELLS RESISTANT TO HERBICIDAL GLUTAMINE SYNTHETASE INHIBITORS.**

(30) Priority: 01.10.84 US 656488
10.06.85 US 742846

(43) Date of publication of application: 12.11.86 Bulletin 86/46

(45) Publication of the grant of the patent: 30.01.91 Bulletin 91/05

(84) Designated Contracting States: AT BE CH DE FR GB IT LI LU NL SE

(56) References cited:
WO-A-84/02913
WO-A-84/02920

J. CELL. BIOCHEM. vol. 0, no. 8, part B, 1984, page 67, abstract 1015; H.M. GOODMAN et al.: "Herbicide resistance in plants: an example of gene amplification"

J. CELL. BIOCHEM., vol. 0, no. 8, part B, 1984, page 148, abstract 1227; H.M. GOODMAN et al.: "Herbicide resistance in plants: an example of gene amplification"

(73) Proprietor: THE GENERAL HOSPITAL CORPORATION
55 Fruit Street
Boston MA 02114 (US)

(72) Inventor: GOODMAN, Howard, M.
10 The Ledges Road
Newton Centre, MA 02159 (US)
Inventor: DONN, Gunter
Sachsenring 35
D-6238 Hofheim (DE)

(74) Representative: Klein, Otto, Dr. et al
Hoechst AG Zentrale Patentabteilung Postfach 80 03 20
D-6230 Frankfurt am Main 80 (DE)

(56) References cited:
Journal of Molecular and Applied Genetics, vol. 2 (1984), pp. 621-635

JOURNAL OF MOLECULAR AND APPLIED GENETICS, 2(6), 1984, Cullimore et al, "Glutamine Synthetase of Phaseolus Vulgaris L. Organ-Specific Expression of a Multigene Family", pp. 589-599, Chemical Abstracts 102: 144097j

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 200 746 B1

Courier Press, Leamington Spa, England.

(56) References cited:
JOURNAL OF MOLECULAR AND APPLIED GENETICS, 2(6), 1984, Donn et al.:"Herbicide-Resistant Alfalfa Cells: An Example of Gene Amplification in Plants", pp. 621-635, Chemical Abstracts 102: 144025j

JOURNAL OF BIOLOGICAL CHEMISTRY, 258(18), September 25, 1983; Young et al, "Mouse 3T6 Cells that Overproduce Glutamine Synthetase", pp. 11260-6

THEORETICAL AND APPLIED GENETICS, 60, 1981; Larkin et al, "Somoclonal Variation - a Novel Source of Variability from Cell Cultures for Plant Improvement", pp. 197-214

FEBS LETTERS, 158(1), July 1983; Cullimore et al, "Glutamine Synthetase from the Plant Fraction of Phaseolus Root Nodules", pp. 107-112

JOURNAL OF BACTERIOLOGY, 155(1), July 1983; Scolnik et al, "The Wild-Type Gene for Glutamine Synthetase Restores Ammonia Control of Nitrogen Fixation to Gln-(glnA) Mutants of Rhodopsendomonas Capsulata", pp. 180-5

EMBO JOURNAL, 3(1), 1984; Sanders et al, "Amplification and Cloning of the Chinese Hamster Glutamine Synthetase Gene", pp. 65-71

JOURNAL OF BIOLOGICAL CHEMISTRY, 258(3), February 1983; Coruzzi et al, "Nucleotide Sequences of Two Pea cDNA Clones Encoding the Small Subunit of Ribulose 1,5-Bisphosphate Carboxylase and the Major Chlorophyll a/b-Binding Thylakoid Polypeptide", pp. 1399-1402

ARCHIVES OF BIOCHEMISTRY AND BIOPHYSICS,202(2), July 1980; Wedler et al, "Interaction of a New Gamma-glutamyl-phosphate Analog, 4-(Phosphonoacetyl)-L-alpha-aminobutyrate, with Glutamine Synthetase Enzymes from Esherichia coli, Plant, and Mammalian Sources", pp. 482-90

## Description

### Technical Field

The present invention relates to the use of recombinant DNA technology for the transformation of plant cells and, more specifically, the design and construction of plant cells which are resistant to herbicidal plant glutamine synthetase inhibitors, such as phosphinothricin.

### Background Art

Glutamine Synthetase (GS) is a plant enzyme which has a central role in the assimilation of ammonia and in the regulation of nitrogen metabolism. Since in most plants glutamine synthetase is, via the glutamine synthethase/glutamate synthase pathway (Fig. 1), the only efficient way to detoxify ammonia released by nitrate reduction, amino acid degradation or photorespiration, plants are very susceptible to potent inhibitors of glutamine synthetase.

One of the most potent glutamine synthetase inhibitors known at present is phosphinothricin (1) (hereinafter PPT):

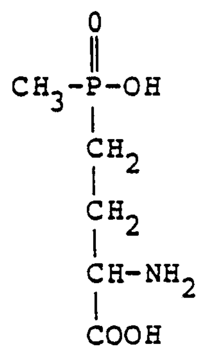

(1)

PPT is a glutamic acid analogue. The compound was initially isolated from a tripeptide antibiotic produced by *Streptomyces viridochromogenes* (Bayer, E. *et al.*, *Helvetica Chimica Acta*, 55: 224 (1972), see also German Patent DOS 2717440, Hoechst, A. G.). PPT is a potent competitive inhibitor of glutamine synthetase from *E. coli* with a $K_i$ of 0.0059 mM.

Schwerdtle, F. (*Zeitschrift fur Pflanzen-Krankheiten und Pflanzenschutz*, IX, 431—440 (1981)) demonstrated that PPT is a non-selective foliar herbicide for the control of undesirable mono and dicotyledonous plants in orchards, vineyards, plantations with minimum tillate, direct drilling, and as a harvest aid. Field trials in West Germany, Spain, South Africa, U.S.A. and Japan showed that most dicotyledonous weeds were well controlled. For monocotyledonous weeds were somewhat higher quantities were needed for good control. Leason, M. *et al.*, (*Phytochemistry*, Volume 21: 855—857 (1982)) demonstrated that PPT is a mixed competitive inhibitor of pea leaf glutamine synthetase with an apparent $K_i$ value of 0.073 mM.

It would be of great interest to be able to confer resistance to PPT, as well as to other GS inhibitors to selected plants, since herbicidal selectivity is quite crucial in any commercially useful herbicide. PPT, as indicated, is non-selective.

There is some precedent for the existence of glutamine synthetases resistant to other compounds. It is known that methionine sulfoximine (MSO), another glutamate analogue, is a mixed competitive inhibitor ($K_i$ value of 0.16mM) of pea leaf glutamine synthetase (Leason, M., *et al.*, *Phytochemistry* 21:855—857 (1982)). Miller, E. S. and Brenchley, J. E. (*The Journal of Biological Chemistry* 256:11307—11321 (1981)) studied the properties of several mutants of Salmonella resistant to MSO. One mutation apparently altered glutamine synthetase at the ammonia binding domain, conferring MSO resistance. More recently, Young and Ringold (*ibid* 258: 11260—11266 (1983)) have reported that mouse 3T6 cells grown in the presence of MSO developed resistance thereto. MSO resistant cells had mRNA enriched for glutamine synthetase, and the authors suggested that this observation implied an amplification of the gene. See also Sanders and Wilson, *EMBO. J.*, 3:65—71 (1984). Neither the Miller, Young, nor Sanders studies were reported on plant GS.

Further, prior to the present invention no studies have been reported on attempts to confer resistance to herbicidal GS inhibitors, such as PPT, by manipulating the glutamine synthetase genes in plant cells.

It would therefore be desirable to develop plant cells which are resistant to herbicidal inhibitors of GS, such as PPT, by manipulating the plant glutamine synthetase genes in said cells. In such manner, it would be possible to confer herbicidal selectivity to any given plant.

### Disclosure of the Invention

The present invention arose out of the discovery that resistance to PPT in plants can arise due to overproduction of glutamine synthetase, a phenomenon which, in the initial experiments, was shown to be due to an underlying gene amplification mechanism. Upon applying selective pressure on certain plant cells in tissue culture, it was possible to isolate PPT resistant strains. The resistance, however, was not due

to the presence of a structural mutant of glutamine synthetase which had less affinity for PPT, but rather, due to gene amplification and concomitant increased concentrations of the enzyme in the plant cells. Out of these initial observations arose the concept of the invention of developing plant cells which overproduce glutamine synthetase (and thus show herbicidal GS-inhibitor resistance), either by gene amplification or by other, different, mechanisms than gene amplification.

The invention is therefore based on producing a plant cell which is resistant to a herbicidal glutamine synthetase (GS) inhibitor, wherein said resistance is caused by plant cell levels of GS activity which, when present in an otherwise herbicidal GS-inhibitor sensitive plant cell, render said cell substantially resistant to said herbicidal GS inhibitor.

The invention can be accomplished by a variety of methods and thus encompasses various embodiments. For example, in one embodiment, the invention is based on producing a plant cell carrying a gene combination comprising:

A) a first DNA sequence coding for a glutamine synthetase (GS) functional in said plant cell, operably linked to

B) a second DNA sequence capable of increasing the levels of expression of said first sequence such that when said combination is present in an otherwise herbicidal GS-inhibitor sensitive plant cell, said cell is substantially resistant to said herbicidal GS-inhibitor.

The invention also comprises a gene combination as described, present in the genome or in a replicating extrachromosomal element of a plant species, which species is heterologous for said first or second DNA sequence of for both.

In another embodiment, the invention is based on producing a herbicidal GS-inhibitor resistant plant cell which contains significantly increased levels of GS activity by virtue of having a significantly larger number of copies of the wild GS gene than the corresponding herbicidal GS-inhibitor sensitive plant cell. In this embodiment, a plant can be obtained by inducing GS gene amplification by selective pressure in cell culture to obtain a resistant variant or strain, followed by growth and development thereof into a callus and full grown plant, and sexual reproduction thereof. Alternatively, a plant can be obtained by inducing GS gene amplification by selection in cell culture of a resistant donor variant or strain, followed by protoplast fusion of the donor cells with an appropriate acceptor cell to yield a morphogenetic hybrid capable of further growth and development.

As another alternative, a resistant plant cell which contains a significantly larger number of copies of the wild gene than the corresponding sensitive cell can be obtained by introducing multiple expressible copies of the wild GS gene into an appropriate extrachromosomal element capable of replication, or into the genome of the plant cell itself. This can be accomplished by transformation at the cell culture stage or at the whole plant stage. Stable multicopy organelles carrying the GS wild gene can also be used to introduce significantly larger number of the GS gene into a cell.

The invention further comprises herbicidal GS inhibitor-resistant, transformed plant cells *per se*, which in their otherwise untransformed state would be herbicidal GS inhibitor sensitive. Whole plants which are herbicidal GS inhibitor resistant are also included.

The invention also comprises intermediate vehicles capable of serving as transformation vectors for plant cells carrying genetic information as described, capable of conferring herbicidal GS inhibitor-resistance, and methods of conferring resistance to plant cells.

The invention further comprises a method of plant control achieved by contacting herbicidal GS inhibitor sensitive plants with plant controlling amounts of a herbicidal GS inhibitor, while in the presence of, and simultaneously contacting plants made resistant to said inhibitor by the methods described.

## Brief Description of the Drawings

The invention will be better understood by reference to the accompanying Figures where

Figure 1 shows the glutamine synthetase/glutamate synthase cycle, wherein it is shown that GS catalyzes the formation of glutamine from glutamic acid and ammonia in a reaction driven by the hydrolysis of ATP to ADP and inorganic phosphate. The amide nitrogen of glutamine provides the source of nitrogen for many biosynthetic reactions, indicating a central role for GS in nitrogen metabolism. The herbicidal GS inhibitor, by inhibiting GS, prevents the biosynthesis of glutamine, thereby preventing ammonia detoxification.

Figure 2 shows the growth characteristics of a wild type (PPT-sensitive) alfalfa cell line in the absence (○) and in the presence of 25 (△), 50 (●) and 100 (□) um L—PPT.

Figure 3 shows the growth characteristics of a variant PPT resistant alfalfa cell line in the absence (○) and in the presence of 100 (△), 200 (●) and 500 (□) um L—PPT.

## Best Mode of Carrying out the Invention

In the description that follows, a number of terms used in recombinant DNA, plant genetics technology and in the present invention are extensively utilized. In order to provide a clear and consistent understanding of the specification and claims, including the scope to be given such terms, the following definitions are provided:

*Nucleotide*. A monomeric unit of DNA or RNA consisting of a sugar moiety, a phosphate, and a nitrogenous heterocyclic base. The base is linked to the sugar moiety via the glycosidic carbon (1′ carbon of

the pentose). The combination of a base and a sugar is called a nucleoside. Each nucleotide is characterized by its base. The four DNA bases are adenine (A), guanmine (G), cytosine (C) and thymine (T). The four RNA bases are A, G, C and uracil (U).

*Genetic sequence.* A linear array of nucleotides connected one to the other by phosphodiester bonds between the 3' and 5' carbons of adjacent pentoses.

*Functional Genetic Sequence.* A genetic sequence coding for a polypeptide having desired activity, regardless of whether the sequence is shorter or longer than that of the full length sequence for the polypeptide. It is also referred to as "functional gene."

*Codon or Triplet.* A DNA sequence of three nucleotides which encodes through mRNA an amino acid, a translation start signal or a translation termination signal. For example, Codons TTA, TTG, CTT, CTC, CTA and CTG encode for the amino acid leucine. TAG, TAA, and TGA are translation stop signals, and ATG is a translation start signal.

*Reading frame.* The grouping of codons during translation of mRNA into an amino acid sequence. During translation, the proper reading frame must be maintained. For example, the sequence GCTGGTTGTAAG may be translated into three reading frames or phrases depending on whether one starts with G, with C, or with T, and thus may yield three different peptide products.

Two sequences are in *operable linkage* when the reading frame of one sequence is linked to the other so that both operate in combination as if they would be present independently.

*Transcription.* The process of producing mRNA from a functional gene.

*Translation.* The process of producing a polypeptide from mRNA.

*Expression.* The process, starting with a functional gene, to produce its polypeptide, being a combination of transcription and translation.

*Cloning vehicle.* A plasmid, phage DNA, or other DNA sequences which are able to replicate in a host cell, which are characterized by one or a small number of endonuclease recognition sites at which such DNA sequences may be cut in a determinable fashion without loss of an essential biological function of the DNA, and which contain a marker suitable for use in the identification of transformed cells. A typical example is an antibiotic resistance marker. The word "vector" is sometimes used for a cloning vehicle.

*Expression vehicle.* A vehicle, analogous to a cloning vehicle, which is particularly useful for production in host cells of a polypeptide by expression of the functional gene coding for said polypeptide, present in the vehicle.

*Replication vehicle.* A cloning or expression vehicle.

*Phage or bacteriophage.* A bacterial virus which may consist of DNA sequences encapsulated in a protein envelope or coat.

*Plasmid.* A non-chromosomal double stranded DNA sequence comprising an intact "replicon," such that the plasmid is replicated in or incorporated into the genome of a host cell. When the plasmid is placed within a unicellular or multicellular organism, the characteristics of that organism may be changed or transformed as a result of the DNA of the plasmid. For example, a plasmid carrying the gene for kanamycin resistance transforms a cell previously sensitive to kanamycin into one which is resistant to it.

*Cloning.* The process of obtaining a population of organisms or DNA sequences derived from one such organism or sequence by asexual reproduction.

*Expression control sequence.* A genetic sequence that controls and regulates expression of functional genetic sequences when operably linked to those sequences. They include sequences known to control the regions which regulate that expression. They comprise both promoter and terminator sequences.

*Plant Promoter.* An expression control sequence which is capable of causing the expression in said plant of any homologous or heterologous genetic sequences or sequences operably linked to such promoter.

*Overproducing Plant Promoter (OPP).* A plant promoter capable of causing the expression in a transformed plant cell of any operably linked functional genetic sequence or sequences to levels (measured by mRNA or polypeptide quantities) which are substantially higher than the levels naturally observable in host cells not transformed with said OPP.

*Degeneracy.* An informational property according to which each amino acid in nature can be coded by more than one codon. For example, leucine can be coded by TTG, TTA, CTA, CTT, CTC or CTG.

By *degenerative variations* as used in the present application and claims, is meant any variation of the polynucleotide fragments of the invention due to the degeneracy of the genetic code. For example, as long as the resulting material still codes for functional GS, or portions thereof, such material is designed to be included in the present invention.

*Glutamine Synthetase (GS).* The definition of this enzyme is functional, and includes any glutamine synthetase capable of functioning in a given desired plant to transform glutamic acid to glutamine in the GS cycle. The term therefore includes not only the enzyme from the specific plant species involved in the genetic transformation, but may include GS from other plant species or microbes or even other eukaryotes, if such GS is capable of functioning in the transformed plant cells. The terms include proteins or polypeptides having more or less than the total structural length of natural plant GS, such as functional partial fragments of GS, or their analogues.

*Phosphinothricin (PPT).* The compound of formula (1), *supra*, in its biologically active form. It may be

the L-, or D- or D,L- forms, and may be alone or in combination with other inert or active compounds which do not interfere with PPT activity.

The invention comprises at its most fundamental level a plant cell which is resistant to a herbicidal glutamine synthetase inhibitor wherein the resistance is caused by levels of GS activity which, when present in an otherwise herbicidal GS inhibitor-sensitive plant cell, render the cell substantially resistant to the herbicidal GS inhibitor.

The terms "herbicidal glutamine synthetase inhibitor" are meant to include any inhibitor, competitive or noncompetitive, that significantly decreases the glutamine synthetase activity of a plant cell of a given species and, as a consequence thereof, causes herbicidal effects in the plant cell. The herbicide resistant plant cell or whole plant survives without irreversible damage a herbicidal GS inhibitor concentration which is lethal for wild type individuals of the same species. Normally, a five-fold or higher increase in herbicidal GS inhibitor resistance is considered significant. However, this number varies from plant species to plant species, and from herbicide to herbicide. It is therefore not feasible to provide a single level for all of the several plant species and herbicides covered by the present invention. Such level, however, can be readily ascertained by those skilled in the art.

Among the herbicidal GS inhibitors covered by the invention are phosphinotricin, methionine sulfoximine, as well as other glutamic acid analogues.

The glutamine synthetase may or may not be from the specific plant cell being transformed. All that is necessary is that the genetic sequence for the enzyme be expressed, and produce, a functional enzyme in the final plant cell. Thus, the invention comprises plant cells containing either homologous GS genes or heterogolous GS genes (and their respective expression products). Broadly, the enzyme might also be that of other plant species, or even enzymes from different organisms, suchas microorganisms or animals. Preferred are plant glutamine synthetase genes and their expression products. Of particular interest are glutamine synthetase genes from the particular plant species which serves as the host in the genetic manipulation, i.e., homologous GS genes. One such glutamine synthetase gene utilizable in the rDNA molecules of the invention is illustrated in Example 2.

Any plant cell which is sensitive to herbicidal GS inhibitors, which is capable of undergoing genetic manipulation by the genetic constructs or methods of the invention, and is capable of expressing said constructs, can be used in the present invention. Among dicotyledonous plants are included various species of potato (Solanum tuberosum); tomato (Lycopersicon esculentum); pepper (Capsicum annumm); tobacco (Nicotiana tabacum); various species of Brassica, especially rapeseed (Brassica napus), various legumes, for example alfalfa (Medicago sativa), clover (Trifolium spec.), soybean (Glycine max), groundnut (Arachis hypogaea), various species of beans (Phaseolus spec., Vicia spec., Vigna spec), peas (Pisum sativum), root crops as beets (Beta vulgaris), carrots (Daucus carota) and sweet potatoes (Ipomea batatus).

There are a variety of embodiments encompassed in the broad concept of the invention. The herbicidal GS-inhibitor resistant plant cell can contain significantly higher levels of GS activity by any of a variety of mechanisms and/or genetic constructs.

For example, the invention comprises, in one of its embodiments, a combination of two genetic sequences: (a) a first genetic sequence coding for a glutamine synthetase functional in a given plant cell, operably linked downstream from (b) a second genetic sequence capable of increasing the levels of gene product of said first sequence such that when the combination is present in an otherwise herbicidal GS-inhibitor sensitive plant cell, said cell is substantially resistant to said herbicidal GS-inhibitor.

The second genetic sequence may be a promoter or an enhancer sequence. If a promoter, it may be a GS promoter or a promoter of another structural gene. In either of the latter two events, the promoter useful in the combination is an overproducing plant promotr (OPP). The only essential characteristic of such promoter is that, when in operable linkage with the genetic sequence for glutamine synthetase, it be capable of promoting expression of said glutamine synthetase to levels such that when the combination is present in an otherwise herbicidal GS-inhibitor sensitive plant cell, the cell is substantially resistant to said GS-inhibitor. Thus, the choice of what plant promoter to use is ruled by functional considerations. The plant promoter will be heterologous or homologous to the host cell. The native, endogenous promoter of said host cell would be incapable of causing resistance by overproduction of GS, since such native promoter normally promotes expression of GS to levels which are so low as to be substantially or completely inhibited by normally used plant-controlling herbicide concentrations. Thus, under one embodiment of the present invention, the native promoter is replaced by an OPP.

Among useful OPP's are included the promoter of the small subunit (ss) of the ribulose bi-phosphate carboxylase, and of the chlorophyll a/b binding protein. The expression of these two genes has been shown to be light induced at the transcriptional level in green tissue (see, for example, *Genetic Engineering of Plants, An Agricultural Perspective*, A. Cashmore, Plenum, New York 1983, pages 29—38, Coruzzi G. *et al.*, *The Journal of Biological Chemistry*, 258: 1399 (1983), or Dunsmuir, P., *et al.*, *Journal of Molecular and Applied Genetics*, 2:285 (1983)).

The invention extends to any plant cell modified according to the methods described, or modified by any other methods which yield herbicidal GS-inhibitor resistance. The plant cell may be alive or not, by itself, in tissue culture or as part of a multicellular plant or a part thereof. Such a multicellular plant, which in its untransformed state is herbicidal GS-inhibitor sensitive, would be resistant when its cells are resistant according to the invention.

Parts obtained from the plant, such as flowers, seeds, leaves, branches, fruit and the like are also covered by the invention, as long as these parts comprise herbicidal GS-inhibitor resistant cells, as noted. In particular, plant parts may be alive or not. Thus, a genetically modified tomato, carrot or tobacco leaf obtained from a resistant tomato, carrot or tobacco plant is included in the invention, even if separate from the plant of origin.

Process of Production and Intermediates used therein

Various methodologies are applicable to carry out the different embodiments of the invention. For example, if the herbicidal GS-inhibitor resistance is brought about by providing significantly increased copies of a structural GS gene, (as opposed to the previously mentioned overproduction of the wild gene) available methodologies include selection for a cell which is resistant by virtue of GS gene amplification, or, alternatively, introduction of multiple copies of the GS gene into the genome or into replicating extrachromosomal element.

Selection is carried out in an appropriate culture medium by cultivating plant cells in the presence of stepwise increase of the herbicidal GS-inhibitor in the medium. After a given period of time has elapsed, such as, for example, a few weeks to several months, it is possible to select a cell population which is several-fold more resistant to the herbicidal GS-inhibitor than the original plant cells. For example, when the cells are alfalfa and the inhibitor is PPT, it has been possible to obtain a PPT-resistant alfalfa cell line after one year of stepwise PPT increases, which line was twenty-fold more resistant to PPT than the original line. When the resistant line was subcultured for several months in the absence of the inhibitor, a slow decline in the percentage of resistant cells was observed in plating experiments on inhibitor-containing agar media, but after more than six months it was still possible to reselect highly resistant cell clones. This was never possible by plating wild-type cells.

Regeneration of calli and grown plant from tissue culture cells is known to those skilled in the art, and it varies from species to species. See, for example, Sheperd, *Scientific American*, 1982. Generally, a suspension of protoplasts containing multiple copies of the GS gene or containing the genetic sequence combination is first provided. Embryo formation can then be induced from the protoplast suspensions, to the stage of ripening and germination as natural embryos. The culture media will generally contain various amino acids and hormones such as auxin and cytokinins. It is advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

For the genotypes of some species, such as tobacco, alfalfa, potato, tomato, petunias, soy beans, rapeseed, and some fruit trees and carrots, regeneration has been clearly demonstrated in the prior art. Such regeneration is well within the skill of the art if the appropriate genotype and history is chosen by screening. Generally, after about more than one year of subculturing, the efficiency of plant regeneration decreases from a given cell culture. Therefore, regeneration is normally most successful if it is initiated shortly after the provision of an appropriate resistant cells culture.

Alternatively, and especially useful in cell cultures which are older than several months or one year, the amplified GS gene can be carried into a regenerative system by crossing and/or fusion. See, for example, Cocking, *et al.*, *Nature*, 293:265 (1981). In this method, a herbicidal GS-inhibitor resistant cell line is provided by selection, and a culture suspension of rapidly dividing cells is obtained by methods well known to those of skill in the art. Protoplasts are isolated therefrom, and preferably irradiated with an amount of radiation and for a time sufficient to inactivate, if not completely irreversibly damage, the host cells. For example, x-ray radiation of 20 Krads can be used advantageously. After irradiating the donor protoplasts, the same are fused with acceptor protoplasts from appropriate susceptible cells. Various methods exist to obtain fusions, such as, for example, polyethylene glycol fusion, high calcium treatment, combinations of both, or even recently, electrofusion. After fusion has occurred, selective growth of the fusion product can be carried out since the fusion product should be herbicidal GS-inhibitor resistant. In particular, if a donor is used which is no longer morphogenetic (i.e., the history of the donor culture is such that several months or upwards of a year may have already elapsed since the original selection), then a simple selection is herbicidal GS-inhibitor containing media can be carried out.

Another method of introducing genetic material into plant cells is to infect a wounded leaf of the plant with transformed *A. tumefaciens* bacteria. Under appropriate growth conditions a ring of calli forms around the wound. The calli are then transferred to growth medium, allowed to form shoots, roots and develop further into plants. Alternatively, grafting onto whole plants can also be done.

An alternative method to introduce multiple copies of the GS gene into the plant cell is by self-ligating a copy of a functional GS (genomic or cDNA) structural gene, utilizing methods well known to those of skill in recombinant DNA technology. The structural genes, each containing its individual promoter, are operably linked to each other by means of appropriate linkers, and 10—30 copies of each gene can in certain instances be introduced into an appropriate replicable expression vehicle, such as a Ti plasmid. Alternatively, the genetic material can be micro-injected directly into plant embryo cells. In the case of monocotyledonous plants, pollen may be transformed with total DNA or an appropriate functional clone providing resistance, and the pollen then utilized to produce progeny by sexual reproduction.

Of course, any other methods utilizable to increase GS activity in a given cell to such levels as will make

the cell herbicidal GS-inhibitor resistant can be utilized. Of particular interest in this invention is the operable linkage of a genetic sequence coding for a structural GS gene to another genetic sequence capable of overproduction of the gene product derived therefrom. This linkage of genetic sequences can be introduced into appropriate plant cells, for example, by means of the Ti plasmid.

The introduction of genetic material into plant cells, especially by use of the so-called tumor inducing (Ti) plasmid of *Agrobacterium tumefaciens*, is a reproducible and predictable technology (See, for example, Caplan, A. *et al.*, "Introduction of Genetic Material into Plant Cells," *Science*: 815—821 (November, 1983); Schell, J. and Van Montagu, M., "The Ti Plasmid as Natural and as Practical Gene Vector for Plants," *Bio/Technology*: April 1983, pps. 175—180; Horsch *et al.*, "Inheritance of Functional Foreign Genes in Plants," *Science*: 233, 496—498 (1984); Fraley, R. T., *et al.*, *Proc. Nat. Acad. Sci. USA*: 80, 4803 (1983), Watson, *et al* ., *Recombinant DNA. A Short Course*, Scientific American Books, 1983, pp. 164—173; and Old and Primrose, *Principles and Gene Manipulation*, 2d Ed., U. Cal. Press, 1981, pp. 138—156.

Ti plasmids contain two regions essential for the production of transfomed cells. One of these, named transfer DNA (T DNA), induces tumor formation. The other, termed virulent region, is essential for the formation but not maintenance of tumors. Transfer DNA, which transfers to the plant genome, can be increased in size by the insertion of the multiply linked GS genes or of the gene combination of the invention without its transferring ability being affected. By removing the tumor causing genes so that they no longer interfere, the modified Ti plasmid can then be used as a vector for the transfer of the gene contructs of the invention into an appropriate plant cell. The foreign DNA to be inserted is usually introduced between the terminal sequences flanking the T-region.

A particularly useful Ti plasmid vector is pGV3850, a non-oncogenic derivative of the nopaline Ti plasmid C58. (See Caplan, *et al.*, *supra*). This vector utilizes the natural transfer properties of the Ti plasmid. The internal T-DNA genes that determine the undifferentiated crown gall phenotype have been deleted and are replaced by any commonly used cloning vehicle (such as pBR 322). The cloning vehicle sequence contained between T-DNA border regions serves as a region of homology for recombination to reintroduce foreign DNA cloned in a derivative of the same cloning vehicle. Any gene construct of the invention cloned in such plasmid can thus be inserted into pGV 3850 by a single recombination of the homologous sequences. Antibiotic resistance markers can be added to the plasmid to select for the recombination event. Herbicide resistance can of course be also used concomitantly or independently. The presence of the nopaline synthase (*nos*) gene is this vector makes it easy to monitor the efficiency of transformation using pGV 3850. A callus in tissue culture can then be tested for the presence of nopaline.

After transformation of the plant cell or plant, the same may be selected by aid of an appropriate marker, such as antibiotic resistance, or more relevant, herbicide resistance, and then grown in conventional ways. In tobacco, protoplast cultures three to five days after the protoplast isolation are suitable for the transformation by Agrobacteria harboring the appropriate Ti-plasmid which contains in its T-DNA region the hybrid GS-gene described. After two days of cocultivation of the protoplast borne cells and the Agrobacteria, the plant cells can be washed by centrifugation and resuspended on fresh medium several times. This removes most of the bacteria. The remaining bacteria are killed by a suitable antibiotic, for example, cefotaxime (400—1,000 ug/ml), added to the protoplast culture medium. The cells are cultivated on nonselective media until they form visible cell aggregates (calli). Then they are plated on media containing the proper herbicide concentration which kills all wild type cells. Only the transformants which express the incorporated GS-hybrid gene survive and continue to grow. These calli can be easily induced to regenerate shoots when transferred to Murashige and Shoog-medium containing 1 mg/l 6-benzyladenine and 0.1 mg/l naphthalene acetic acid. The shoots can be rooted on hormone free MS-medium or directly on perlite or vermiculite, and transferred to pots. The plantlets are ready to grow in the greenhouse and can be tested for herbicide resistances.

Other systems, such as cauliflower mosaic virus, CaMV (Hohn, B. *et al.*, in "Molecular Biology of Plant Tumors," *Academic Press*, New York 1982 pps. 549—560; and *Howell*, United States Patent 4,407,956) can also be used. The entire CaMV viral DNA genome is inserted into a parent bacterial plasmid creating a recombinant DNA molecule which be propagated in bacteria. After cloning, the recombinant plasmid is cleaved with restriction enzymes either at random or unique sites in the viral portion of the recombinant plasmid for insertion of the gene combination of the invention. A small oligonucleotide, described as a linker, having a unique restriction site may also be inserted. The modified recombinant plasmid again may be cloned and further modified by introduction of larger pieces of a gene construct into the unique restriction site of the linker. The modified viral portion of the recombinant plasmid is then excised from the parent bacterial plasmid, and used to incoculate the plant cells or plants. This virus is described in the aforementioned *Howell* patent as being particularly good for insertion of genes capable of enhanced production of protein, greater tolerance to stress, resistance to pest and pesticides, nitrogen fixation, and the like.

Normally, the desired GS sequences are operably linked *in vitro* to each other or to an overproducing promoter, by known recombinant methodology. For example, the structural gene for GS, normally the genomic version thereof, is separated from its normal promoter by restriction on the region between such promoter and the initiation AUG codon. A transciptional fusion with, e.g., the small subunit of the ribulose bi-phosphate carboxylase is then carried out. The contruct is then inserted into an appropriate restriction site of the plant vehicle.

For example, when using CaMV DNA as a vehicle, the genetic construct is inserted into a site or sites in the viral DNA, without destroying infectivity of the viral DNA or its movement throughout the plant. Thus, the construct can be inserted into a variety of restriction sites, cloning the product and determining whether the essential characteristics of the virus have been retained. In this manner, one can rapidly isolate a relatively large amount of modified virus which can be screened for infectivity and movement. After the viral portion of the hybrid DNA plasmid has been modified, the modified virus may be excised from the hybrid DNA plasmid, and may be used to inoculate plants directly in linear form or ligated in circles.

Various techniques may be employed for infecting plant cells with CaMV vehicles. Young leaves may be mildly abraded and then contacted with the viral DNA. After infection, the viral DNA may be transmitted by aphids, where the aphid transmissible gene is operative. Mechanical techniques can also be employed. Alternatively, tissues or single cells may be infected.

Uses

The use of vehicles containing the gene constructs of the invention is as intermediates in the preparation of whole herbicide-resistant plant cells and plants. Thus, not only the plant cells made resistant according to the methods of the invention, but also all of the vehicles or vectors, derive their utility from the utility of the final product, the whole plant.

The utility for a whole plant made herbicide resistant according to the invention is obvious. Such a plant, when brought into contact with otherwise plant controlling or suppressing amounts of herbicide, would be resistant thereto. This would allow herbicide treatment to be selective for any desired plant or group of plants.

In addition, the resistance to herbicide would enable its use as a selectable marker in the transfer of other genes to the plant, or cells thereof.

By the terms "plant controlling amounts of herbicidal GS-inhibitor" is meant to include functionally, an amount of herbicide which is capable of affecting the growth or development of a given plant. Thus, the amount may be small enough to simply retard or suppress the growth or development, or the amount may be large enough to irreversibly destroy the sensitive plant. Normally, most dicotylendonous plants and weeds may be controlled at rates of between 0.5 to 1.5 kg/ha ai. For monocotyledonous plants, rates between 0.5 kg/ha ai, and up to about 2.0 kg/ha ai are normally used. The herbicide can, of course, be contacted with the appropriate plant using well known spraying or spreading methods. For example, foliar administration used in the prior art for control of weeds by PPT can be used with PPT-resistant plants falling within the invention.

The invention also encompasses a method of plant control which comprises contacting a herbicidal GS-inhibitor sensitive plant cell or plant, with plant controlling amounts of herbicidal GS-inhibitor, wherein the contact is carried out while the sensitive plant cell or plant is present simultaneously with or among the herbicidal GS-inhibitor resistant plant cells or plants of the invention. Thus, foliar herbicidal treatment of plants in a field or cultivar, which plants include both those comprising herbicide resistant plant cells and those comprising herbicide sensitive plant cells, and wherein both are simultaneously contacted with the herbicidal GS inhibitor during the treatment operation, is a method included in the present invention.

Having now generally described this invention, the same will become better understood by reference to certain specific examples which are included herein for purposes of illustration only and are not intended to be limiting unless otherwise specified.

Example 1

Isolation and Characterization of PPT-Resistant Alfalfa Cell Culture

An alfalfa cell line which is able to grow in the presence of 3 mM L-phosphinothricin in the culture medium was selected. Alfalfa was chosen since it is one of the model plants in which the regeneration of a whole plant from single isolated cells or protoplasts can be readily achieved.

The selection of the phosphinothricin resistant alfalfa line was done by stepwise increase of the inhibitor concentration in the liquid culture medium. Within 6 months a cell population was selected which was at least 20-fold more resistant to phosphinothricin than the original cell line. Measuring the packed cell volume of the suspension cultures revealed that wild type cells were completely inhibited by $2.5 \times 10^{-5}$M L-phosphinothricin. The resistant cells grew as well in the presence of $5 \times 10^{-4}$M of the compound as in its absence (Figures 2 and 3).

When the protein patterns of crude cell extracts of the susceptible and resistant cell lines were compared by SDS-polyacrylamide gel electrophoresis, the overproduction of a polypeptide in the molecular weight range 40—42 KD could be observed in the PPT-resistant cell line. Purified GS of alfalfa has the same molecular weight. The enzyme activity of the cell extracts was determined in parallel. The specific GS-activity in the resistant cells was 5—10 fold higher than in wild type cells. The specific activity of GS in the PPT-resistant cell lines is in the same order of magnitude as in root nodule tissue of alfalfa, which, as all nodule tissues of efficiently dinitrogen fixing legumes, has high GS-activity. Measurements of the GS activity in the presence of various amounts of phosphinothricin revealed the same degree of enzyme inhibition by PPT in both cell types, suggesting that no structural change of the GS-protein had occured during the selection of the resistant variant.

Messenger RNA was isolated from wild type and resistant cells using a guanidinium isothiocyanate RNA-extraction method, followed by the separation of the mRNA through an oligo dT-cellulose column. 4

microgram mRNA could be isolated per gram of cell material. *In vitro* translation of the isolated mRNA from both cell lines yielded patterns of polypeptides similar to the *in vivo* pattern obtained in $^{35}S$-methionine labelled extracts. This indicated that the isolated mRNA was fairly undegraded. While the *in vivo* labelled proteins showed a striking difference in the amount of the 40—42 KD polypeptide, the difference between wild type and variant cell line in the *in vitro* translated protein patterns was not pronounced.

First and second strand cDNA were synthersized from the mRNA population of the PPT-resistant variant. After $S_1$-cleavage and poly C-tailing of the cDNA, the DNA was annealed to poly G-tailed pBR 322 vector DNA. *E. coli* strain MC1061 was transformed with the recircularized vector.

3.5 × $10^4$ colonies were obtained per mg of annealed vector DNA. 80% of the colonies were ampicillin (amp) sensitive, and 50% of the amp sensitive clones had inserts which could be detected by Pst digestion of the plasmids. 10% of the inserts were longer than 1,000 base pairs.

1,800 colonies were grown on nitrocellulose filters and the filters were probed with $^{32}P$-labelled first strand cDNA probe having increased percentage of GS-specific sequences. Amongst such clones it was expected to find a GS-specific cDNA clone. A GS-cDNA clone for a *Phaseolus* cDNA library prepared from mRNA of root nodules was obtained from an outside source. This GS-cDNA clone enabled the identification of one alfalfa cDNA clone in the cDNA library, which hybridized strongly to the *Phaseolus* GS-DNA.

The insert DNA of the alfalfa GS-cDNA clone was sequenced. The alfalfa and the *Phaseolus* GS-cDNAs were then used as probes to characterize the difference between the wild alfalfa cells and the variant GS-overproducing cells.

Total genomic DNA was prepared from alfalfa leaves, from wild-type cell line and from three sublines of the resistant cell line which had been selected in presence of 6 × $10^{-4}$M, 2 × $10^{-3}$M and 3 × $10^{-3}$M L-phosphinothricin. The DNA was digested with BamHi, EcoRI, and HindIII, and a combination of 2 enzymes. After agarose gel electrophoresis of the digested DNAs, the DNA fragments were transferred onto nitrocellulose filters by Southern blotting. The filterbound DNA was hybridized with $^{32}P$-labelled c-DNA inserts of the alfalfa and the *Phaseolus* GS-clones. DNA of the same size hybridized with both probes. One predominant band hybridizes with the same intensity in all 5 DNA samples with the alfalfa probe. A second band, which is barely visible in the wild type DNA digest, strongly hybridizes with DNA of the resistant cell lines. The highest degree of hybridization was observed with DNA of the highly resistant cell lines, indicating amplification of a DNA fragment during the development of the resistance. The alfalfa cDNA probe strongly hybridized to the amplified DNA fragment and, to a lesser extent, to the non-amlified DNA with GS homology.

In order to confirm the reason why the variant alfalfa cell line had become resistant to PPT, both Northern and Southern blots were repeated using wild type and variant cell lines. The Northern blot indicated that there is an increase of about 8 fold in glutamine synthetase mRNA from wild type. When Southern blots were done using genomic DNA from both cell lines, there was a clear indication of gene duplication of glutamine synthetase in the mutant alfalfa cell line. The duplication seems to be 5 to 15 fold above the unduplicated glutamine synthetase gene as estimated by hybridization. It also appeared to be an exact duplication, that is, only one band increased in hybridization by 7 to 11 fold.

## Example 2
## Isolation and Characterization of Glutamine Synthetase from the PPT-Resistant Cell Culture

### Materials and Methods

#### CNBr Cleavage

Glutamine synthetase (12 nmol) was dissolved in 1 mL of 70% formic acid, 5 mg CNBr was added, and the cleavage proceeded for 24 hr at room temperature (Gross, Meth. Enzymol., 11:238—255 (1967)). After dilution with distilled water (6 mL), the mixture was lyophilized twice.

#### High Performance Liquid Chromatography of CNBr Peptides

Reverse-phase high performance liquid chromatography (HPLC) was carried out on an Ultrapore RPSC (0.46 ID × 7.5 cm) at 40°C using 0.1% trifluoroacetic acid (TFA) (Bennet *et al., Biochem. J., 168*:9—13 (1977)) and a linear gradient of 0—60% acetonitrile (30 min; flow rate, 0.5 mL/min). The CNBr peptides were dissolved in the minimum volume of 6 M guanidine hydrochloride in 0.1 TFA. Chromatographic peaks from several separations were collected manually, pooled, and lyophilized. Detection was at 214 nm using a Beckman 160 detector.

#### Amino acid analysis

Amino acid composition were determined after acid hydrolysis of samples in sealed, evacuated tubes (Pyrex®, culture, rimless, 6 × 50 mm) at 110°C for 24 hr in constant boiling HCl (0.025 mL) (Moore, *Chemistry and Biology of Peptides*, Ann Arbor Science, Ann Arbor, Michigan, 629—653 (1972)). Using a Beckman 6300 amino acid, analyzer equipped with two Hewlett-Packard 3390A integrators, ninhydrin and two channel (440 and 570 nm) integration provided analysis of all amino acids, except tryptophan, cysteine,

asparagine, and glutamine, with confidence values of 1—7% at 100 pmol/amino acid, and with lower limit of detection of 25 pmol. Analyses were done 3—5 times on each sample and background controls.

Protein sequence analysis:

Automated Edman degradation was performed with an Applied Biosystems 470A sequencer. The sequence program was developed earlier for the gas-liquid solid phase peptide and protein sequence (Hewick *et al.*, *J. Biol. Chem.*, *256*: 7990—7997 (1981)). The program contains one coupling step (44°C, 26 min) and a single cleavage step (44°C, 6.7 min). Automated conversion of the 2-anilino-5-thiazolinone derivatives (Pth-amino acids) were 25% trifluoroacetic acid (50°C, 33 min). Polybrene® (1.5 mg) (Tarr *et al.*, *Anal. Biochem.*, *84*:622—627 (1978); Klapper *et al.*, *ibid*, *85*:126—131 (1978)) was added to the glass filter disc in the cartridge prior to degradation of protein or peptides, and five sequence cycles were run to reduce contaminants derived from Polybrene®. Angiotensin II (1 nmol) was added to the cartridge filter, and ten degradative cycles were completed. Sequencing of angiotensin allowed an assessment of the chemical and mechanical operation of the sequencer prior to the sequencing of an unknown. Protein sequence analysis of sperm whale apomyoglobin (100—200 pmol) routinely demonstrated an initiated yield of 45—55%, average repetitive yield of 92—93%, and an average lag per cycle of 2—3%. All Pth-amino acids were identified by reverse-phase high performance liquid chromatography on a cyano column (0.45 × 25 cm) using a 15 mM sodium acetate buffer (pH 5.5), and a complex gradient of acetonitrile/methanol (92.5:7.5 v/v), based on a system developed by Hunkapiller and Hood, *Methods Enzymol.*, *91*:486—493 (1983). This HPLC system separated the Pth-amino acids and internal standard (methyl ester of Pth-Glu) from the major contaminants: a dithio-threitol-adduct, N-dimethyl-N'-phenylthiourea, and diphenylthiourea. Methionine and proline were not routinely separated, but a modification of the gradient separated these two Pth-amino acids. Internal standard (200 pmol) was added to each cycle collection, and the samples were dried in a Speedvac Concentrator with an RH100—6 rotor. The dried samples were dissolved in 0.025 mL of water/acetonitrile (80:20, v/v), and an aliquot (0.017 mL) (68% of total sample) injected automatically. The Pth-amino acids were detected by UV absorbance at 254 nm using a Beckman 160 detector.

Purification Scheme

The process of purification is briefly summarized in the following scheme:

100 gm. frozen cells
Add 50 mM Tris-pH 7.5
↓
Use blender low speed (30 seconds)
High speed (2 minutes)
Spin 8000 rpm, 5°, 10 minutes
↓
Supernatant 7.5% v/v 1% Protamine Sulfate, 15 minutes, 4°
↓
Spin 8000 rpm, 5° 10 minutes
↓
Supernatant 1.5% v/v 1M $MgSO_4$ pH to 6.8 with 1M acetic acid β-mercaptoethanol to 7 mM
↓
Bind to hydroxyapatite, 4°, 15 minutes
↓
Wash hydroxyapatite 5X with 50 mM Tris pH 7.5
0.28M $MgSO_4$
10% ethylene glycol
↓
Elute GS 5X with 50 mM Tris pH 7.5
0.5M $MgSO_4$
16.7% ethylene glycol
↓
Pool eluted fractions, dialyze against 10 mM Tris
pH 6.8
10 mM $MgSO_4$
↓
Sephadex® G—100 column, pool activity peak

100 grams of frozen variant alfalfa tissue culture cells were mixed with dry ice and ground to a fine powder in a blender. After thawing, the mixture was spun at 8,000 rpm at 4° for 20 minutes. Protamine sulfate precipitation was carried out on the supernatant to remove nucleic acid. The $MgSO_4$ concentration was brought to 10 mM, the β-mercaptoethanol to 7 mM, and the pH adjusted to 6.8 with 1M acetic acid. The crude extract was bound to 1,2 hydroxyapatite. The 1,2 hydroxyapatite was washed batchwise 5 times with

10 mM Tris, pH 7.5, 0.28M $MgSO_4$, and 10% ethylene glycol. Glutamine synthetase was eluted by bringing the $MgSO_4$ concentration to 0.5M. Five batch elutions were done. After dialyzing against 10 mM Tris, pH 7.5, 10 mM $mgSO_4$, it was loaded onto a Sephadex® G—200 column. Fractions with good glutamine synthetase activity were pooled. By SDS acrylamide gel electrophoresis and silver staining, the protein was better than 95% pure. An amino acid composition showed the composition of alfalfa glutamine synthetase to be nearly the same as that of a published composition from soy bean (Table 1)

Table 1

Amino Acid Composition of Alfalfa Glutamine Synthetase

| Amino Acid | Soy Bean* Root (mole%) | Pea Leaf (mole %) | Alfalfa (mole % + C.V.) |
|---|---|---|---|
| Ala | 8.4 | 6.9 | 8.7 (±0.4) |
| Arg | 5.6 | 4.0 | 5.0 (±0.5) |
| Asx | 10.4 | 10.7 | 11.4 (±0.6) |
| Cys | 0.8 | N.D | N.D |
| Glx | 10.0 | 10.1 | 9.9 (±0.5) |
| Gly | 10.6 | 19.1 | 13.5 (±0.7) |
| His | 2.2 | 3.6 | 3.1 |
| Ile | 6.6 | 5.6 | 8.4 (±0.4) |
| Leu | 6.7 | 6.5 | 7.6 (±0.9) |
| Lys | 5.9 | 5.7 | 6.8 (±0.3) |
| Met | 1.6 | N.D. | 0.6 (±0.3) |
| Phe | 2.8 | 2.6 | 2.3 |
| Pro | 6.1 | 8.8 | 8.0 (±0.6) |
| Ser | 5.4 | 7.8 | 6.5 (±0.4) |
| Thr | 5.1 | 4.9 | 5.6 (±0.3) |
| Trp | 1.5 | N.D. | N.D. |
| Tyr | 3.9 | 0.5 | 3.4 (±0.7) |
| Val | 6.3 | 4.8 | 4.4 (±1.5) |

* R. H. McParland, *et al., Biochem. J.* (1976) *153*:597—606
N.D. = None Deleted
C.V. = Confidence Values (S.D./mean)

Sequencing

A first attempt at sequencing was initiated using the purified native protein. There was no obtainable sequence, indicating that the $NH_2$-terminal was naturally blocked. A CNBr cleavage was carried out, and the fragments were separated by HPLC. The amino acid composition of the protein indicated that there were only two to five methionines. Two of the HPLC separated CNBr fragments yielded useful sequence information.

Sequencing of purified glutamine synthetase fragment was then carried out. The results are as follows:

Arg-Glu-Asp-Gly-Gly-Tyr-Glu-Val-Ile-
Leu-Lys-Ala-Ile-Glu-Lys-Leu-Gly-Lys-
Lys-(Glu/His)-Lys-Glu-His-Ile-Ala-
Ala-Tyr-Gly-Gly-Gly-Asn

This sequence corresponds to part of the sequence obtained from the complete variant alfalfa cDNA clone. The deduced protein sequence is shown on the following page.

```
1    ATG TCT CTC CTT TCA GAT CTT ATC AAC CTT GAC CTC TCC GAA ACC ACC GAG AAA ATC ATC GCC GAA TAC ATA TGG    75
     Met Ser Leu Leu Ser Asp Leu Ile Asn Leu Asp Leu Ser Glu Thr Thr Glu Lys Ile Ile Ala Glu Tyr Ile Trp
76   ATT GGT GGA TCT GGT TTG GAC TTG AGG AGC AAA GCA AGG ACT CTA CCA GGA CCA GTT ACT GAC CCT TCA CAG CTT    150
     Ile Gly Gly Ser Gly Leu Asp Leu Arg Ser Lys Ala Arg Thr Leu Pro Gly Pro Val Thr Asp Pro Ser Gln Leu
151  CCC AAG TGG AAC TAT GAT GGT TCC AGC ACA GGT CAA GCT CCT GGA GAA GAT AGT GAA GTT ATT ATC TAC CCA CAA    225
     Pro Lys Trp Asn Tyr Asp Gly Ser Ser Thr Gly Gln Ala Pro Gly Glu Asp Ser Glu Val Ile Ile Tyr Pro Gln
226  GCC ATT TTC AAG GAC CCA TTT AGA AGG GGT AAC AAT ATC TTG GTT ATG TGT GAT GCA TAC ACT CCA GCT GGA GAG    300
     Ala Ile Phe Lys Asp Pro Phe Arg Arg Gly Asn Asn Ile Leu Val Met Cys Asp Ala Tyr Thr Pro Ala Gly Glu
301  CCC ATT CCC ACC AAC AAG AGA CAT GCA GCT GCC AAG ATT TTC AGC CAT CCT GAT GTT GTT GCT GAA GTA CCA TGG    375
     Pro Ile Pro Thr Asn Lys Arg His Ala Ala Ala Lys Ile Phe Ser His Pro Asp Val Val Ala Glu Val Pro Trp
376  TAT GGT ATT GAG CAA GAA TAC ACC TTG TTG CAG AAA GAC ATC AAT TGG CCT CTT GGT TGG CCA GTT GGT GGT TTT    450
     Tyr Gly Ile Glu Gln Glu Tyr Thr Leu Leu Gln Lys Asp Ile Asn Trp Pro Leu Gly Trp Pro Val Gly Gly Phe
451  CCT GGA CCT CAG GGA CCA TAC TAT TGT GGA GCT GGT GCT GAC AAG GCA TTT GGC CGT GAC ATT GTT GAC TCA CAT    525
     Pro Gly Pro Gln Gly Pro Tyr Tyr Cys Gly Ala Gly Ala Asp Lys Ala Phe Gly Arg Asp Ile Val Asp Ser His
526  TAC AAA GCC TGT CTT TAT GCC GGC ATC AAC ATC AGT GGA ATC AAT GGT GAA GTG ATG CCT GGT CAA TGG GAA TTC    600
     Tyr Lys Ala Cys Leu Tyr Ala Gly Ile Asn Ile Ser Gly Ile Asn Gly Glu Val Met Pro Gly Gln Trp Glu Phe
601  CAA GTT GGT CCC TCA GTT GGT ATC TCT GCT GGT GAT GAG ATA TGG GTT GCT CGT TAC ATT TTG GAG AGG ATC ACT    675
     Gln Val Gly Pro Ser Val Gly Ile Ser Ala Gly Asp Glu Ile Trp Val Ala Arg Tyr Ile Leu Glu Arg Ile Thr
676  GAG GTT GCT GGT GTG GTG CTT TCC TTT GAC CCA AAA CCA ATT AAG GGT GAT TGG AAT GGT GCT GGT GCT CAC ACA    750
     Glu Val Ala Gly Val Val Leu Ser Phe Asp Pro Lys Pro Ile Lys Gly Asp Trp Asn Gly Ala Gly Ala His Thr
751  AAT TAC AGC ACC AAG TCT ATG AGA GAA GAT GGT GGC TAT GAA GTC ATC TTG AAA GCA ATT GAG AAG CTT GGG AAG    825
     Asn Tyr Ser Thr Lys Ser Met Arg Glu Asp Gly Gly Tyr Glu Val Ile Leu Lys Ala Ile Glu Lys Leu Gly Lys
826  AAG CAC AAG GAG CAC ATT GCT GCT TAT GGA GAA GGC AAC GAG CGT AGA TTG ACA GGG CGA CAT GAG ACA GCT GAC    900
     Lys His Lys Glu His Ile Ala Ala Tyr Gly Glu Gly Asn Glu Arg Arg Leu Thr Gly Arg His Glu Thr Ala Asp
901  ATT AAC ACC TTC TTA TGG GGT GTT GCA AAC CGT GGT GCG TCG ATT AGA GTT GGA AGG GAC ACA GAG AAA GCA GGG    975
     Ile Asn Thr Phe Leu Trp Gly Val Ala Asn Arg Gly Ala Ser Ile Arg Val Gly Arg Asp Thr Glu Lys Ala Gly
976  AAA GGT TAT TTC GAG GAT AGG AGG CCA TCA TCT AAC ATG GAT CCA TAT GTT GTT ACT TCC ATG ATT GCA GAC ACC    1050
     Lys Gly Tyr Phe Glu Asp Arg Arg Pro Ser Ser Asn Met Asp Pro Tyr Val Val Thr Ser Met Ile Ala Asp Thr
1051 ACC ATT CTC TGG AAA CCA TAA    1071
     Thr Ile Leu Trp Lys Pro End
```

Preparation of a Functional Genomic
Sequence Coding for Glutamine Synthetase

Total genomic DNA was obtained from the alfalfa tissue culture using a protocol similar to that used to obtain that of *Phaseolus* root nodules (Cullimore, J. D., and Miflin, B. J., *FEBS Letters*, 158:107—112 (1983)), and digested totally with Bam HI. The material was then size fractionated in a potassium acetate 5—20% gradient and separated into aliquots. Each aliquot was then run on a 1% agarose gel and probed in a Southern transfer system with cDNA glutamine synthetase probe obtained from a Pst cut of the coding sequence probe shown above. Positive hybridizations were then selected for fragments of size greater than 4 Kb. These were ligated to a BV—2 lambda vector, 1000,000 phages were plated and probed with the glutamine synthetase cDNA probe as above. Four positive plaques were obtained. These were then grown and plaque-hydridization purified for 3—4 rounds. A purified 4 Kb genomic clone was isolated and religated into M13mp9. This clone was sequenced from both ends. In addition, the clone was also fragmented with Hae III, subcloned into MP—9 and again sequenced.

In this manner, this 4 Kb 5′ fragment (as well as an 8 Kb 3′ fragment also obtained from the digestion of the genomic DNA) can be completely sequenced. Both of these fragments together will provide a complete functional gene, the reading frame being predicted from the protein sequence information at hand, the known GS molecular weight and the relative incidence of stop triplets in the three possible reading frames. With a complete functional sequence for GS at hand, the same can be expressed by any of the methods described previously.

Having now fully described this invention it will be understood that the same can be operated within a broad and equivalent range of structures, products, processes, and uses without affecting the scope of the invention or any embodiment thereof.

**Claims for the Contracting States: BE CH DE FR GB IT LI LU NL SE**

1. A plant cell which is resistant to a herbicidal glutamine synthetase (GS) inhibitor, wherein said resistance is caused by plant cell levels of GS activity which, when present in an otherwise herbicidal GS-inhibitor sensitive plant cell, render said cell substantially resistant to said herbicidal GS-inhibitor, said plant cell carrying a DNA sequence combination which comprises:

a) a first DNA sequence coding for a glutamine synthetase (GS) having the sequence shown on page 13, operably linked to

b) a second DNA sequence capable of increasing the levels of expression of said first DNA sequence such that when such combination is present in an otherwise herbicidal GS-inhibitor sensitive plant cell, said cell is substantially resistant to said herbicidal GS-inhibitor.

2. The plant cell of claim 1 wherein said combination is integrated in the genome of said plant cell.

3. The plant cell of claim 1 wherein said combination is present in a Ti plasmid of Agrobacterium tumefaciens.

4. The plant cell of any of claims 1, 2 or 3 wherein said herbicidal GS-inhibitor is PPT.

5. A DNA sequence combination operable in a given plant cell which comprises:

a) a first DNA sequence coding for a glutamine synthetase having the sequence shown on page 13, operably linked to

b) a second DNA sequence capable of increasing the levels of expression of said first sequence such that when said combination is present in an otherwise herbicidal GS-inhibitor sensitive plant cell, said cell is substantially resistant to said herbicidal GS-inhibitor.

6. The DNA sequence combination of claim 5 wherein said second DNA sequence is a promoter.

7. The DNA sequence combination of any of claims 5 or 6 which in integrated in the genome of said plant cell.

8. The DNA sequence combination of any of claims 5 or 8 which is present in the Ti plasmid of Agrobacterium tumefaciens.

9. The DNA sequence combination of any of claims 5 or 6 wherein said herbicidal GS-inhibitor is PPT.

10. A herbicidal GS-inhibitor resistant plant comprising a cell according to any of claims 1, 2 or 3.

11. The plant of claim 10, wherein said herbicidal GS-inhibitor is PPT.

12. A method of conferring herbicidal GS-inhibitor resistance to a plant cell which comprises transforming said cell with the DNA sequence combination of claim 5.

13. The method of claim 12 wherein said herbicidal GS-inhibitor is PPT.

14. A method of selectively controlling herbicidal GS-inhibitor sensitive plants which comprises contacting a herbicidal GS-inhibitor sensitive plant with plant controlling amounts of the herbicide GS-inhibitor, wherein said contact is carried out while simultaneously contacting a herbicidal GS-inhibitor resistant plant as claimed in claims 10 or 11.

15. The method of claim 14 wherein said herbicidal GS-inhibitor is PPT.

16. A recombinant DNA molecule comprising a DNA sequence coding for glutamine synthetase having the sequence shown on page 13.

17. The molecule of claim 16 which is a vehicle capable of transforming a plant cell.

18. A DNA molecule having the sequence shown on page 13.

**Claims for the Contracting State: AT**

1. A method of making a plant cell resistant to a herbicidal glutamine synthetase (GS) inhibitor, wherein said resistance is caused by plant cell levels of GS activity which, when present in an otherwise herbicidal GS-inhibitor sensitive plant cell, render said cell substantially resistant to said herbicidal GS-inhibitor, which comprises introducing into the said plant cell a DNA sequence combination of:

a) a first DNA sequence coding for a glutamine synthetase (GS) having the sequence shown on page 13, operably linked to

b) a second DNA sequence capable of increasing the levels of expression of said first DNA sequence such that when such combination is present in an otherwise herbicidal GS-inhibitor sensitive plant cell, said cell is substantially resistant to said herbicidal GS-inhibitor.

2. The method of Claim 1 wherein said combination is integrated in the genome of said plant cell.

3. The method of Claim 1 wherein said combination is present in a Ti plasmid of *Agrobacterium tumefaciens*.

4. The method of any of Claims 1, 2 or 3 wherein said herbicidal GS-inhibitor is PPT.

5. A method of preparing a DNA sequence combination operable in a given plant cell which comprises combining

a) a first DNA sequence coding for a glutamine synthetase (GS) having the sequence shown on page 13, operably linked to

b) a second DNA sequence capable of increasing the levels of expression of said first DNA sequence such that when such combination is present in an otherwise herbicidal GS-inhibitor sensitive plant cell, said cell is substantially resistant to said herbicidal GS-inhibitor.

6. The method of Claim 5 wherein said second DNA sequence is a promoter.

7. The method of Claim 5 or 6 wherein the sequence combination is integrated in the genome of said plant cell.

8. The method of Claim 5 or 6 wherein the DNA sequence is present in the Ti plasmid of *Agrobacterium tumefaciens*.

9. The method of Claim 5 or 6 wherein said herbicidal GS-inhibitor is PPT.

10. A method of conferring herbicidal GS-inhibitor resistance to a plant cell which comprises transforming said cell with the DNA sequence combination of Claim 5.

11. The method of Claim 10, wherein said herbicidal GS-inhibitor is PPT.

12. A method of selectively controlling herbicidal GS-inhibitor sensitive plants which comprises contacting a herbicidal GS-inhibitor sensitive plant with plant controlling amounts of the herbicide GS-inhibitor, wherein said contact is carried out while simultaneously contacting a herbicidal GS-inhibitor resistant plant prepared as claimed in Claim 1.

13. The method of claim 12 wherein said herbicidal GS-inhibitor is PPT.

**Patentansprüche für die Vertragsstaaten: BE CH DE FR GB IT LI LU NL NL SE**

1. Pflanzenzelle, die gegenüber einem herbiziden Glutamin Synthetase (GS)-Inhibitor resistent ist, wobei die genannte Resistenz durch die GS-Aktivitätswerte in der Pflanzenzelle verursacht wird, die, wenn sie in einer ansonsten gegenüber einem herbiziden GS-Inhibitor sensitiven Pflanzenzelle vorhanden sind, die genannte Zelle im wesentlichen gegenüber dem genannten herbiziden GS-Inhibitor resistent machen, wobei die genannte Pflanzenzelle eine DNA-Sequenz-Kombination aufweist, umfassend

a) eine erste DNA-Sequenz, die für Glutamin Synthetase (GS) codiert mit der auf Seite 13 gezeigten Sequenz und die operabel an

b) eine zweite DNA-Sequenz, die geeignet ist, die Expressionswerte der genannten ersten DNA-Sequenz zu steigern, so daß wenn eine solche Kombination in einer ansonsten gegenüber einem herbiziden GS-Inhibitor sensitiven Pflanzenzelle vorhanden ist, die genannte Zelle im wesentlichen gegenüber dem genannten herbiziden GS-Inhibitor resistent ist, gekoppelt ist.

2. Pflanzenzelle nach Anspruch 1, wobei die genannte Kombination in das Genom der genannten Pflanzenzelle integriert ist.

3. Pflanzenzelle nach Anspruch 1, wobei die genannte Kombination in einem Ti Plasmid von Agrobacterium tumefaciens vorhanden ist.

4. Pflanzenzelle nach einem der Ansprüche 1, 2 oder 3, wobei der herbizide GS-Inhibitor PPT ist.

5. Eine in einer vorgegebenen Pflanzenzelle operable DNA Sequenz-Kombination, umfassend

a) eine erste DNA-Sequenz, die für Glutamin Synthetase codiert und die auf Seite 13 gezeigte Sequenz aufweist und operabel an

b) eine zweite DNA-Sequenz, die geeignet ist, die Expressionswerte der genannten ersten Sequenz zu steigern, so daß, wenn die genannte Kombination in einer ansonsten gegenüber einem herbiziden GS-Inhibitor sensitiven Pflanzenzelle vorhanden ist, die genannte Zelle im wesentlichen gegenüber dem genannten herbiziden GS-Inhibitor resistent ist, gekoppelt ist.

6. Die DNA-Sequenz-Kombination nach Anspruch 5, wobei die genannte zweite DNA-Sequenz ein Promoter ist.

7. Die DNA-Sequenz-Kombination nach einem der Ansprüche 5 oder 6, wobei sie in das Genom der genannten Pflanzenzelle integriert ist.

8. Die DNA-Sequenz-Kombination nach einem der Ansprüche 5 oder 6, welche in dem Ti Plasmid von Agrobacterium tumefaciens vorhanden ist.

9. Die DNA-Sequenz-Kombination nach einem der Ansprüche 5 oder 6, wobei der herbizide GS-Inhibitor PPT ist.

10. Eine gegenüber einem herbiziden GS-Inhibitor resistente Pflanze beinhaltend eine Zelle nach einem der Ansprüche 1, 2 oder 3.

11. Planze nach Anspruch 10, wobei der genannte herbizide GS-Inhibitor PPT ist.

12. Verfahren zum resistent-Machen einer Pflanzenzelle gegenüber einem herbiziden GS-Inhibitor, wobei die genannte Zelle mit der DNA-Sequenz-Kombination nach Anspruch 5 transformiert wird.

13. Verfahren nach Anspruch 12, wobei der genannte herbizide GS-Inhibitor PPT ist.

14. Ein Verfahren zur selektiven Unterdrückung von gegenüber einem herbiziden GS-Inhibitor sensitiven Pflanzen, wobei eine gegenüber einem herbiziden GS-Inhibitor sensitive Pflanze mit Pflanzenunterdrückenden Mengen des herbiziden GS-Inhibitors in Kontakt gebracht wird und der genannte Kontakt gleichzeitig mit dem in-Kontakt-Bringen einer gegenüber einem herbiziden GS-Inhibitor resistenten Pflanze nach Anspruch 10 oder 11 durchgeführt wird.

15. Verfahren nach Anspruch 14, wobei der genannte herbizide GS-Inhibitor PPT ist.

16. Ein rekombinantes DNA-Molekül, enthaltend eine DNA Sequenz welche für Glutamin Synthetase codiert und die auf Seite 13 gezeigte Sequenz aufweist.

17. Molekül nach Anspruch 16, das ein zum Transformieren einer Pflanzenzelle geeignetes Vehikel ist.

18. DNA Molekül, das die auf Seite 13 gezeigte Sequenz aufweist.

**Patentansprüche für den Vertragsstaat: AT**

1. Verfahren zum resistent-Machen einer Pflanzenzelle gegenüber einem herbiziden Glutamin Synthetase (GS)-Inhibitor, wobei die genannte Resistenz durch GS-Aktivitätswerte in der Pflanzenzelle verursacht wird, die, wenn sie in einer ansonsten gegenüber einem herbiziden GS-Inhibitor sensitiven Pflanzenzelle vorhanden sind, die genannte Zelle im wesentlichen gegenüber dem genannten herbiziden GS-Inhibitor resistent machen, wobei in die genannte Pflanzenzelle eine DNA-Sequenz-Kombination aus

a) einer ersten DNA-Sequenz, die für Glutamin Synthetase (GS) codiert und die auf Seite 13 gezeigten Sequenz aufwiest und die operabel gekoppelt ist an

b) eine zweite DNA-Sequenz, die geeignet ist die Expressionswerte der genannten ersten DNA-Sequenz zu steigern, so daß, wenn eine solche Kombination in einer ansonsten gegenüber einem herbiziden GS-Inhibitor sensitiven Pflanzenzelle vorhanden ist, die genannte Zelle im wesentlichen gegenüber dem genannten herbiziden GS-Inhibitor resistent ist, eingeführt wird.

2. Verfahren nach Anspruch 1, wobei die genannte Kombination in das Genom der genannten Pflanzenzelle integriert wird.

3. Verfahren nach Anspruch 1, wobei die genannte Kombination in einem Ti Plasmid von Agrobacterium tumefaciens vorhanden ist.

4. Verfahren nach einem der Ansprüche 1, 2 oder 3, wobei der herbizide GS-Inhibitor PPT ist.

5. Verfahren zur Herstellung einer DNA-Sequenz-Kombination, die in einer vorgegebenen Pflanzenzelle operabel ist, wobei

a) eine erste DNA-Sequenz, die für Glutamin Synthetase (GS) codiert und die auf Seite 13 gezeigte Sequenz aufweist, operabel an

b) eine zweite DNA-Sequenz, die geeignet ist die Expressionswerte der genannten ersten Sequenz zu steigern, so daß, wenn eine solche Kombination in einer ansonsten gegenüber einem herbiziden GS-Inhibitor sensitiven Pflanzenzelle vorhanden ist, die genannte Zelle im wesentlichen gegenüber dem genannten herbiziden GS-Inhibitor resistent ist, gekoppelt wird.

6. Verfahren nach Anspruch 5, wobei die genannte zweite DNA-Sequenz ein Promoter ist.

7. Verfahren nach Anspruch 5 oder 6, wobei die DNA-Sequenz Kombination in das Genom der genannten Pflanzenzelle integriert ist.

8. Verfahren nach Anspruch 5 oder 6, wobei die DNA-Sequenz in dem Ti Plasmid von Agrobacterium tumefaciens vorhanden ist.

9. Verfahren nach Anspruch 5 oder 6, wobei der herbizide GS-Inhibitor PPT ist.

10. Verfahren zum resistent-Machen einer Pflanzenzelle gegenüber einem herbiziden GS-Inhibitor, wobei die genannte Zelle mit der DNA-Sequenz-Kombination nach Anspruch transformiert wird.

11. Verfahren nach Anspruch 10, wobei der genannte herbizide GS-Inhibitor PPT ist.

12. Verfahren zum selektiven Unterdrücken von gegenüber einem herbiziden GS-Inhibitor sensitiven Pflanzen, wobei eine gegenüber einem herbiziden GS-Inhibitor sensitive Pflanze mit Pflanzenunterdrückenden Mengen des herbiziden GS-Inhibitors in Kontakt gebracht wird und wobei der genannte Kontakt simultan mit dem in-Kontakt-Bringen einer gegenüber einem herbiziden GS-Inhibitor resistenten Pflanze, hergestellt nach Anspruch 1, ausgeführt wird.

15. Verfahren nach Anspruch 12, wobei der genannte herbizide GS-Inhibitor PPT ist.

**Revendications pour les Etats contractants: BE CH DE FR GB IT LI LU NL SE**

1. Cellule végétale qui est résistante à une inhibiteur de glutamine synthétase (GS) herbicide, ladite résistance étant causée par des teneurs de la cellule végétale en activité de la GS, qui, lorsqu'elles eont présentes dans une cellule végétale par ailleurs sensible à un inhibiteur de GS herbicide, rendent ladite cellule pratiquement résistante audit inhibiteur de GS herbicide, ladite cellule végétale comportant une combinaison de séquence d'ADN qui comprend:

a) une première séquence d'ADN codant pout la glutamine synthétase (GS), comportant la séquence représentée page 13, fonctionnellement liée à

b) une seconde séquence d'ADN capable d'accroître les taux d'expression de ladite première séquence d'ADN de sorte que lorsqu'une telle combinaison est présente dans une cellule végétale par ailleurs sensible à un inhibiteur de GS herbicide, ladite cellule est pratiquement résistante audit inhibiteur de GS herbicide.

2. Cellule végétale selon la revendication 1, dans laquelle ladite combinaison est intégrée dans le génome de ladite cellule végétale.

3. Cellule végétale selon la revendication 1, dans laquelle ladite combinaison est présente dans un plasmide Ti d'*Agrobacterium tumefaciens*.

4. Cellule végétale selon l'une quelconque des revendications 1, 2 ou 3, dans laquelle ledit inhibituer de GS herbicide est la phosphinothricine (PPT).

5. Combinaison de séquences d'ADN apte à fonctionner dans une cellule végétale donnée, qui comprend:

a) une première séquence d'ADN codant pour la glutamine synthétase, comportant la séquence représentée page 13, fonctionnellement liée à

b) une seconde séquence d'ADN capable d'accroître les taux d'expression de ladite première séquence de sorte que lorsqu'une telle combinaison est présente dans une cellule végétale par ailleurs sensible à un inhibiteur de GS herbicide, ladite cellule est pratiquement résistante audit inhibiteur de GS herbicide.

6. Combinaison de séquences d'ADN selon la revendication 5, dans laquelle ladite seconde séquence d'ADN est un promoteur.

7. Combinaison de séquences d'ADN selon l'une quelconque des revendications 5 ou 6, qui est intégrée dans le génome de ladite cellule végétale.

8. Combinaison de séquences d'ADN selon l'une quelconque des revendications 5 ou 6, qui est présente dans le plasmid Ti d'*Agrobacterium tumefaciens*.

9. Combinaison de séquences d'ADN selon l'une quelconque des revendications 5 ou 6, dans laquelle ledit inhibiteur des GS herbicide est la PPT.

10. Plante résistante à un inhibiteur de GS herbicide, comprenant une cellule selon l'une quelconque des revendications 1, 2 ou 3.

11. Plante selon la revendication 10, dans laquelle ledit inhibiteur de GS herbicide est la PPT.

12. Procédé pour conférer à une cellule végétale la résistance à un inhibiteur de GS herbicide, lequel comprend la transformation de ladite cellule par la combinaison de séquences d'ADN selon la revendication 5.

13. Procédé selon la revendicaton 12, dans lequel ledit inhibiteur de GS herbicide est la PPT.

14. Procédé pour la maîtrise sélective de la croissance de plantes sensibles à un inhibiteur de GS herbicide, lequel comprend la mise en contact d'une plante sensible à un inhibiteur de GS herbicide avec des quantités, maîtrisant la croissance végétale, de l'inhibiteur de GS herbicide, dans lequel on effectue ledit contact tout en mettant simultanément en contact une plante résistante à un inhibiteur de GS herbicide selon la revendication 10 ou 11.

15. Procédé selon la revendicaton 14, dans lequel ledit inhibiteur de GS herbicide est la PPT.

16. Molécule d'ADN recombinant, comprenant une séquence d'ADN codant pout la glutamine synthétase comportant la séquence représentée page 13.

17. Molécule selon la revendication 16, qui est un vecteur capable de transformer une cellule végetale.

18. Molécule d'ADN comportant la séquence représentée page 13.

**Revendications pour l'Etats contractant: AT**

1. Procédé pour rendre une cellule végétale résistante à une inhibiteur de glutamine synthétase (GS) herbicide, ladite résistance étant causée par des teneurs de la cellule végétale en activité de la GS, qui, lorsqu'elles sont présentes dans une cellule végétale par ailleurs sensible à un inhibiteur de GS herbicide, rendent ladite cellule pratiquement résistante audit inhibiteur de GS herbicide, lequel ccomprend l'introduction dans ladite cellule végétale d'une combinaison de séquence d'ADN constituée de:

a) une première séquence d'ADN codant pour la glutamine synthétase (GS), comportant la séquence représentée page 13, fonctionnellement liée à

b) une seconde séquence d'ADN capable d'accroître les taux d'expression de ladite première séquence d'ADN de sorte que lorsqu'une telle combinaison est présente dans une cellule végétale par ailleurs sensible à un inhibiteur de GS herbicide, ladite cellule est pratiquement résistante audit inhibiteur de GS herbicide.

2. Procédé selon la revendication 1, dans laquelle ladite combinaison est intégrée dans le génome de ladite cellule végétale.

3. Procédé selon la revendication 1, dans lequel ladite combinaison est présente dans un plasmide Ti d'*Agrobacterium tumefaciens*.

4. Procédé selon l'une quelconque des revendications 1, 2 ou 3, dans lequel ledit inhibiteur de GS herbicide est la phosphinothricine (PPT).

5. Procédé de préparation d'un combinaison de séquences d'ADN apte à fonctionner dans une celle végétale donnée, qui comprend la combinaison:

a) d'une première séquence d'ADN codant pour la glutamine synthétase (GS), comportant la séquence représentée page 13, fonctionnellement liée à

b) une seconde séquence d'ADN capable d'accroître les taux d'expression de ladite première séquence d'ADN de sorte que lorsqu'une telle combinaison est présente dans une cellule végétale par ailleurs sensible à un inhibiteur de GS herbicide, ladite cellule est pratiquement résistante audit inhibiteur de GS herbicide.

6. Procédé selon la revendication 5, dans lequel ladite seconde séquence d'ADN est un promoteur.

7. Procédé selon la revendication 5 ou 6, dans lequel la combinaison de séquences d'ADN est intégree dans le génome de ladite cellule végétale.

8. Procédé elon la revendication 5 ou 6, dans lequel la séquence d'ADN est présente dans le plasmid Ti d'*Agrobacterium tumefaciens*.

9. Procédé selon la revendication 5 ou 6, dans lequel ledit inhibiteur des GS herbicide est la PPT.

10. Procédé pour conférer à une cellule végétale la résistance à un inhibiteur de GS herbicide, lequel comprend la transformation de ladite cellule par la combinaison de séquences a'ADN de la revendication 5.

11. Procédé selon la revendication 10, dans lequel ledit inhibiteur de GS herbicide est la PPT.

12. Procédé pour la maîtrise sélective de la croissance de plantes sensibles à un inhibiteur de GS herbicide, lequel comprend la mise en contact d'une plante sensible à un inhibiteur des GS herbicide avec des quantités, maîtrisant la croissance végétale, de l'inhibiteur de GS herbicide, dans lequel on effectue ledit contact tout en mettant simultanément en contact une plante résistante à un inhibiteur de GS herbicide, préparée selon la revendication 1.

13. Procédé selon la revendicaton 12, dans lequel ledit inhibiteur de GS herbicide est la PPT.

*Fig. 1*

*THE GLUTAMATE SYNTHASE CYCLE*

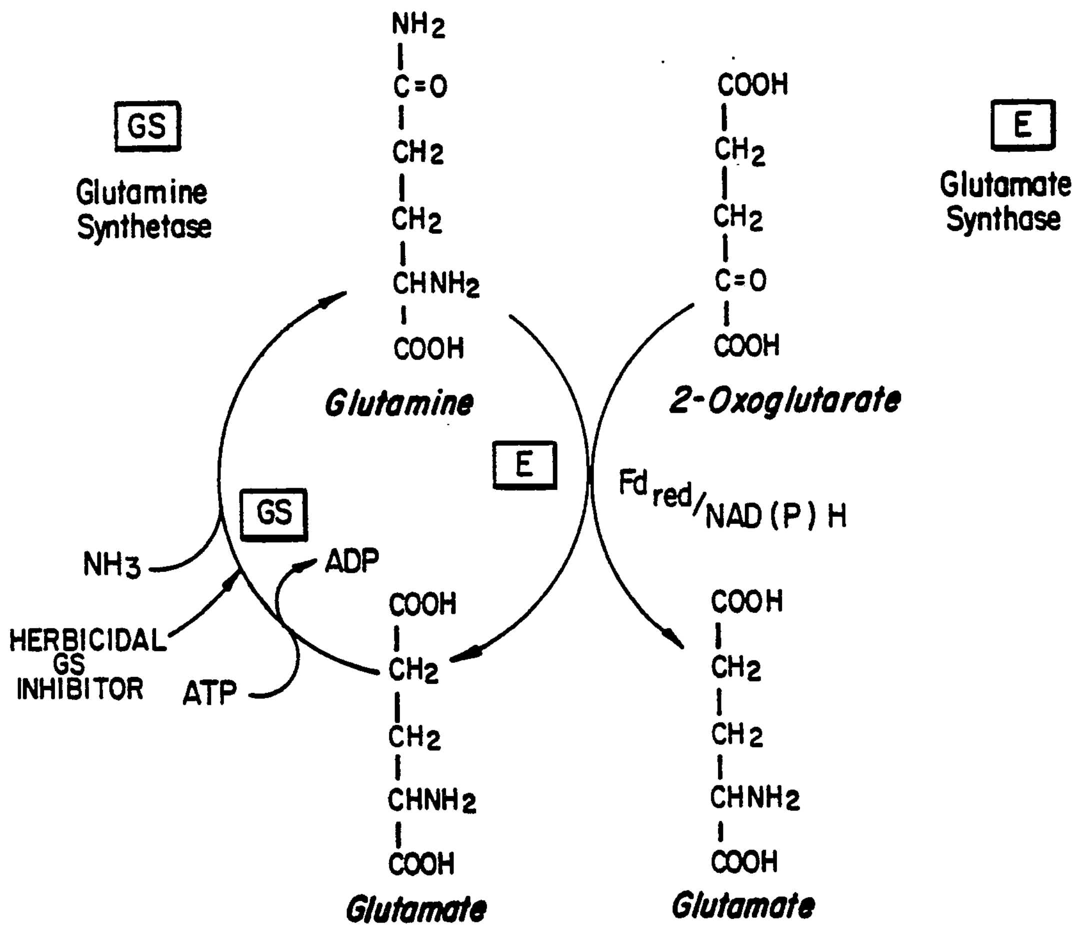

*Fig.2*

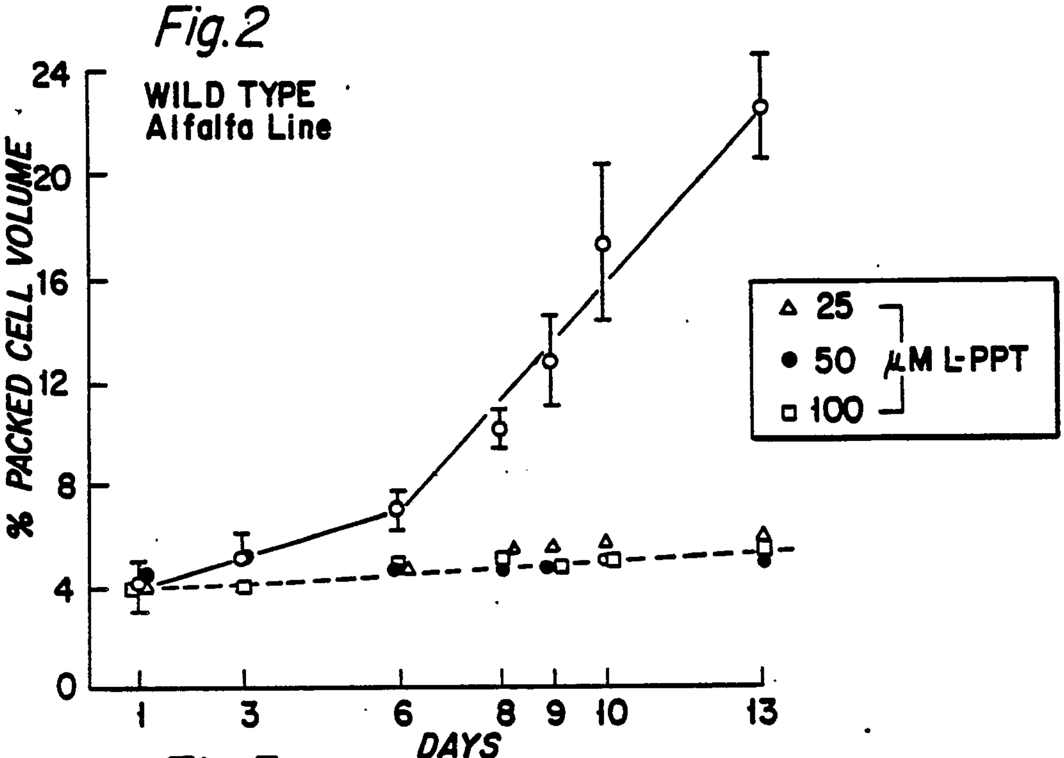
WILD TYPE
Alfalfa Line
% PACKED CELL VOLUME
24
20
16
12
8
4
0
1
3
6
8
9
10
13
DAYS
△ 25
● 50 µM L-PPT
□ 100

*Fig.3*

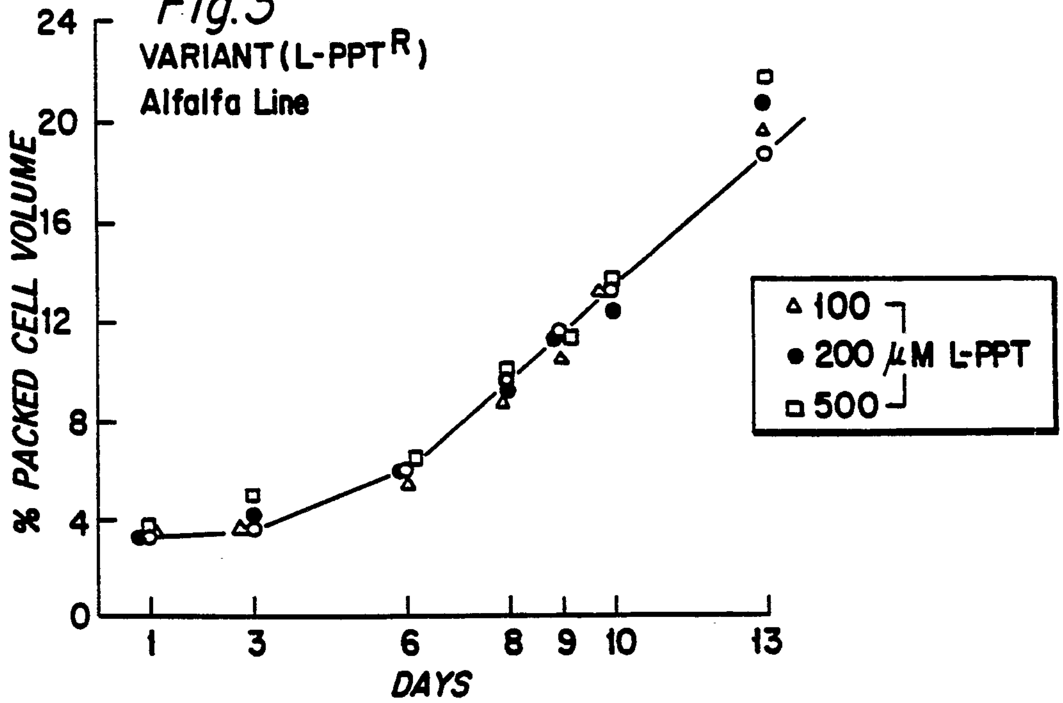
VARIANT (L-PPT^R)
Alfalfa Line
% PACKED CELL VOLUME
24
20
16
12
8
4
0
1
3
6
8
9
10
13
DAYS
△ 100
● 200 µM L-PPT
□ 500